# EUROPÄISCHE PATENTSCHRIFT

(11) **EP 3 269 422 B1**
(45) Veröffentlichungstag und Bekanntmachung des Hinweises auf die Patenterteilung: **18.12.2019**
(21) Anmeldenummer: 17181024.5
(22) Anmeldetag: 12.07.2017
(51) Int. Cl.: A61N 1/372, A61N 1/378, H04B 5/00

(54) **ENERGIE- UND DATENÜBERTRAGUNGSVORRICHTUNG UND VERFAHREN ZUM BETREIBEN EINER SOLCHEN**
ENERGY AND DATA TRANSMISSION DEVICE AND METHOD FOR OPERATING SAME
DISPOSITIF DE TRANSFERT DE DONNÉES ET D'ÉNERGIE ET SON PROCÉDÉ DE FONCTIONNEMENT

(30) Priorität: 12.07.2016 DE 102016212626
(43) Veröffentlichungstag der Anmeldung: 17.01.2018
(73) Patentinhaber: Dualis Medtech GmbH, 82229 Seefeld (DE)
(72) Erfinder: Hornung, Heinz, 82229 Seefeld (DE); Schwarzbach, Stefan, 82234 Weßling (DE); Habersetzer, Christian, 82211 Herrsching (DE); Schuster, Dominik, 82234 Weßling (DE); Michel, Sören, 82229 Seefeld (DE); Sommer, Christoph, 82319 Starnberg (DE)
(74) Vertreter: dompatent von Kreisler Selting Werner - Partnerschaft von Patent- und Rechtsanwälten mbB

(56) Entgegenhaltungen:
- GB-A- 2 297 037
- US-A- 4 681 111
- US-B2- 6 772 011

## Beschreibung

Die Erfindung betrifft eine Energieübertragungsvorrichtung zur drahtlosen Übertragung von Energie zu einem aktiven Implantat. Die Erfindung betrifft ferner ein Verfahren zum Betreiben einer solchen Energieübertragungsvorrichtung.

Es ist aus dem Stand der Technik bekannt, aktive medizinische Implantate drahtlos mit Energie zu versorgen. Hierzu wird eine extrakorporale Sendespule induktiv mit einer implantierten Empfangsspule gekoppelt.

Bei der induktiven drahtlosen Energieübertragung wird häufig eine Datenübertragung vom Energieempfänger zum Energiesender benötigt. Beispielsweise kann diese verwendet werden, um Informationen zur Regelung der Energieübertragung oder andere Informationen über den Status des Empfängers zu übertragen.

Die Regelungsinformationen sind von besonderer Wichtigkeit, wenn die Positionierung zwischen der Sendespule und der Empfangsspule nicht genau bestimmbar ist. Dies ist häufig bei medizinischen Implantaten der Fall, beispielsweise wenn der Patient atmet. In einer solchen Situation ändert sich die induktive Koppelung zwischen der Sendespule und der Empfangsspule, so dass die Eigenschaften der Übertragungsstrecke nicht genau bekannt sind und sich sogar kurzfristig ändern können. Die Regelung muss dann sehr häufig (beispielsweise zehn- oder fünfzigmal pro Sekunde) anhand der Statusinformationen des Energieempfängers die Parameter des Energiesenders anpassen.

Bei medizinischen Implantaten kann eine Datenübertragung vom Implantat nach außen auch sicherheitskritisch sein.

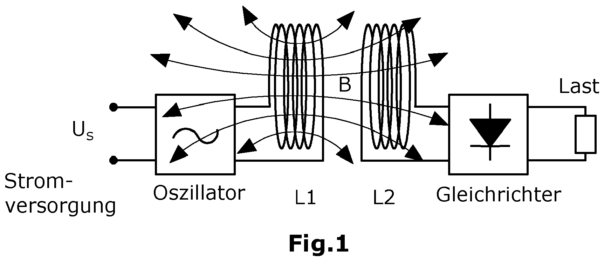

In Fig. 1 ist die grundsätzliche Funktionsweise einer drahtlosen Energieübertragung an ein Implantat dargestellt. Eine Stromversorgung ist mit einem Oszillator verbunden, über den der Sendespule L1 eine Wechselspannung zugeführt wird. Diese induziert in der Empfangsspule L2 eine Wechselspannung, die einem Gleichrichter zugeführt wird. Hierdurch kann die Last, nämlich das Implantat, mit elektrischer Energie versorgt werden.

Verschiedene Verfahren für eine Energieübertragung vom Energieempfänger zum Energiesender sind in den folgenden Veröffentlichungen beschrieben:
[1] Islam, Ashraf Bin: "Design of Wireless Power Transfer and Data Telemetry System for Biomedical Applications", PhD Diss., University of Tennessee, 2011
[2] http://www.low-powerdesign.com/article TI-Qi.html
[3] Wireless Power Consortium: "System Description Wireless Power Transfer", Vol. I, Part 1, Version 1.1.2, June 2013
[4] https://en.wikipedia.org/wiki/Powermat Technologies
[5] Rezence Alliance for Wireless Power: "A4WP Wireless Power Transfer System, Baseline System Specification (BSS)", V 1.2.1, Final Approved Specification, May 07, 2014

Aus dem Stand der Technik ist das "Load Shift Keying (LSK) Verfahren" bekannt, bei dem die Last auf der Empfängerseite abhängig von den zu übertragenden Daten geändert wird. Die Änderung der Last kann über eine zusätzliche resistive oder kapazitive Last erfolgen.

In Veröffentlichung [1] wird ein LSK-Verfahren beschrieben, bei dem das Load Shift Keying durch eine zusätzliche resistive Last erzeugt wird.

In Veröffentlichungen [2] und [3] wird ein Verfahren beschrieben, bei dem das Load Shift Keying mit einer kapazitiven Last erzeugt wird.

Eine weitere Vorrichtung, bei der ein Load Shift Keying-Verfahren beschrieben wird, ist in Veröffentlichung [4] beschrieben.

Die Vorrichtung gemäß Veröffentlichung [5] nutzt einen separaten 2,4 GHz Funkkanal.

Ein separater Funkkanal erhöht die Anzahl der erforderlichen Bauteile für die Schaltung und verursacht eine erhöhte Störanfälligkeit. Dies ist gerade bei medizinischen Implantaten unerwünscht. Die notwendige Antenne erhöht den Bauraum zusätzlich. Eine redundante Auslegung bei sicherheitskritischen Anwendungen kann ferner zwei verschiedene Funkkanäle erfordern, was weiteren zusätzlichen Aufwand bedeutet.

Ein separater Funkkanal kann sich prinzipbedingt stärker störend auf andere Geräte auswirken bzw. leichter selbst gestört werden.

Das LSK-Verfahren ist prinzipbedingt auf Datenraten deutlich unterhalb der Frequenz der Energieübertragung beschränkt. Gerade bei schlecht bestimmbarem Übertragungskanal muss die Last relativ deutlich verändert werden, damit ein auswertbares Signal beim Energiesender ankommt. Dies wiederum erzeugt beträchtliche Verluste, die bei einem medizinischen Implantat besonders nachteilig sind.

US 6,772,011 B2 beschreibt eine Vorrichtung zum Übertragen von Informationen zwischen einer extrakorporalen Vorrichtung und einem Implantat, das durch eine induktive Kopplung mit Energie versorgt wird.

US 4,681,111 A beschreibt ebenfalls eine solche Vorrichtung.

Aufgabe der Erfindung ist es, eine Energieübertragungsvorrichtung zur drahtlosen Übertragung von Energie und/oder Daten von und/oder zu einem aktiven Implantat bereitzustellen, die einen vereinfachten Aufbau aufweist und eine zuverlässige Funktion gewährleistet. Weiterhin ist es Aufgabe der Erfindung, ein Verfahren zum Betreiben einer solchen Energieübertragungsvorrichtung bereitzustellen.

Die Aufgabe wird durch die unabhängigen Ansprüche gelöst.

Alle im Folgenden genannten Beispiele und Ausführungen, die nicht unter die unabhängigen Ansprüche fallen, sind nicht Teil der Erfindung.

Die erfindungsgemäße Energieübertragungsvorrichtung dient der drahtlosen Übertragung von Energie zu einem aktiven Implantat. Unter einem aktiven Implantat wird ein solches Implantat verstanden, das für seinen Betrieb eine Energie benötigt, die ihm extern zugeführt wird. Hierbei kann es sich beispielsweise um einen Herzschrittmacher, eine Herzunterstützungsvorrichtung (Ventricular Assist Device), künstliche Muskeln etc. handeln.

Die erfindungsgemäße Vorrichtung weist eine Sendespule auf, die mit einer Energiequelle elektrisch verbindbar ist. Bei der Energiequelle kann es sich beispielsweise um eine Batterie handeln. Die Vorrichtung umfasst ferner eine implantierbare Empfangsspule, die zur drahtlosen Energieübertragung induktiv mit der Sendespule koppelbar ist. Der Sendespule wird eine Wechselspannung zugeführt, die ein sich veränderndes Magnetfeld erzeugt. Dieses induziert in der Empfangsspule wiederum eine Wechselspannung, die zum Betrieb des aktiven Implantats verwendet werden kann. Diese Wechselspannung kann durch einen Gleichrichter in eine Gleichspannung umgewandelt werden. Erfindungsgemäß ist eine implantierte Primärspule der erfindungsgemäßen Vorrichtung elektrisch mit einem Modulator verbunden, wobei durch den Modulator ein Modulieren einer der implantierten Primärspule zugeführten Wechselspannung gemäß einem Datensignal erfolgt, so dass eine Datenübertragung von der implantierten Primärspule zur extrakorporalen Sekundärspule erfolgt. Die Frequenz der Datenübertragung ist von der Frequenz, mit der die Energie von der Sendespule zur Empfangsspule übertragen wird, verschieden.

Erfindungsgemäß erfolgt ein Übertragen von Informationen betreffend die Energieregelung der von der Sendespule zu übertragenden Energie durch ein pulsweitenmoduliertes Signal von der Primärspule zur Sekundärspule. Zusätzlich erfolgt ein Übermitteln anderer Informationen, die nicht die Energieregelung betreffen, durch eine Frequenzmodulation der Trägerfrequenz des pulsweitenmodulierten Signales oder durch eine Modulation der Frequenz, mit der die Pulse des pulsweitenmodulierten Signales gesendet werden.

Durch die genannten Merkmale ist es möglich, eine einfache und sichere Datenübertragung vom Implantat in Richtung der externen Vorrichtung bereit zu stellen. Die extrakorporale Sendespule kann anhand der Informationen zur Energieregelung die von ihr bereitgestellte Leistung regeln. Dies kann beispielswese anhand des Duty Cycle des pulsweitenmodulierten Signals erfolgen. Weiterhin ist es möglich, andere Informationen, die nicht die Energieregelung betreffen, von der implantierten Vorrichtung in Richtung der extrakorporalen Vorrichtung zu senden, ohne dass hierfür eine zusätzliche Antenne bzw. Sendevorrichtung verwendet werden müsste, die über diejenige hinausgeht, die für die Energieregelung verwendet wird.

In bevorzugter Ausführungsform ist die implantierte Primärspule die Empfangsspule. Zusätzlich oder alternativ kann die extrakorporale Sekundärspule die Sendespule sein. Anders ausgedrückt kann die bereits vorhandene Empfangsspule als implantierte Primärspule und die bereits vorhandene Sendespule als extrakorporale Sekundärspule verwendet werden, um den beschriebenen Kommunikationskanal für die Energieregelung und die anderen Informationen, die nicht die Energieregelung betreffen, bereitzustellen. In dieser Ausführungsform wird somit die Empfangsspule, deren ursprüngliche Funktion es ist, Energie von der Sendespule zu empfangen, dazu verwendet, Daten an die Sendespule zu senden. Es sind somit für die Datenübertragung keine zusätzlichen Komponenten zur eigentlichen Signalübertragung erforderlich. Dadurch dass die Frequenz der Datenübertragung von der Frequenz der Energieübertragung verschieden ist, kann sichergestellt werden, dass sich die beiden Übertragungsarten nicht gegenseitig beeinflussen. Wird eine geeignete Frequenz für die Datenübertragung verwendet, so kann eine hohe Datenrate bei gleichzeitig niedrigen Verlusten sichergestellt werden. Die Verwendung eines separaten Funkkanals ist nicht erforderlich. Wie im weiteren Verlauf der vorliegenden Anmeldung beschrieben, können für die Datenübertragung elektrische Komponenten verwendet werden, die ohnehin größtenteils bereits vorhanden sind.

Das erfindungsgemäße Übertragen von Daten erfolgt bevorzugt über das Nahfeld.

Es ist bevorzugt, dass der Modulator durch einen Übertrager induktiv mit der implantierten Primärspule gekoppelt ist. Bei dem Übertrager handelt es sich bevorzugt um eine Überträger-Primärspule, die elektrisch mit dem Modulator verbunden ist, und eine Überträger-Sekundärspule, die elektrisch mit der Empfangsspule verbunden ist.

Es ist bevorzugt, dass die Frequenz der Datenübertragung höher ist als die Frequenz, mit der die Energie von der Sendespule zur Empfangsspule übertragen wird und von vorhandenen Störern möglichst weit entfernt liegt, wie z.B. von den Oberwellen der Energieübertragung.

Es ist auch möglich, dass für die Datenübertragung eine Frequenz verwendet wird, die niedriger als die Frequenz für die Energieübertragung ist. Dies stellt jedoch wegen der niedrigeren verfügbaren Datenrate eine weniger vorteilhafte Alternative dar.

In einer bevorzugten Ausführungsform ist ein zweiter Übertrager vorgesehen zum induktiven Auskoppeln des von der Sendespule empfangenen Datensignals aus einer mit der extrakorporalen Sekundärspule verbundenen elektrischen Leitung. Dieser zweite Übertrager weist bevorzugt eine Überträger-Primärspule auf, die mit der extrakorporalen Sekundärspule elektrisch verbunden ist. Diese ist induktiv mit einer Sekundärspule gekoppelt, die elektrisch mit einer Auswertschaltung verbunden ist.

Die Auswertschaltung weist einen Bandpassfilter auf, der lediglich die Nutzfrequenz der Datenübertragung passieren lässt. Der Bandpassfilter kann einen Vorfilter aufweisen, der die Energieübertragungsfrequenz soweit unterdrückt, dass der Hauptfilter nicht übersteuert wird. Der Bandpassfilter kann passiv oder aktiv (mit Verstärkung) sein.

Dem Bandpassfilter nachgeschaltet kann ein Verstärker sein, der das hochfrequente Datensignal auf ein für die Demodulation geeignetes Level anhebt. Nach dem Verstärker kann ein Demodulator angeordnet sein, der die Daten aus dem Datensignal extrahiert.

In einer weiteren bevorzugten Ausführungsform ist ein zweiter Modulator vorgesehen, der elektrisch mit der extrakorporalen Sekundärspule verbunden ist. Dieser dient dem Modulieren einer der extrakorporalen Sekundärspule zugeführten Wechselspannung gemäß einem von der extrakorporalen Sekundärspule zur implantierten Primärspule zu übertragenden zweiten Datensignal. Hierdurch ist es möglich, zusätzlich zum Datensignal von der implantierten Primärspule zur extrakorporalen Sekundärspule ein Datensignal in umgekehrter Richtung, d.h. von der extrakorporalen Sekundärspule zur implantierten Primärspule zu senden. Zusätzliche technische Komponenten außer dem genannten Modulator sind hierzu nicht notwendig.

Bevorzugt ist die für die Datenübertragung von der extrakorporalen Sekundärspule zur implantierten Primärspule verwendete Frequenz verschieden von der für die Datenübertragung von der implantierten Primärspule zur extrakorporalen Sekundärspule verwendeten Frequenz. Alternativ kann bei Verwendung der gleichen Frequenz zu unterschiedlichen Zeiten in unterschiedliche Richtungen gesendet werden. Des Weiteren können alternativ verschiedene Modulationsverfahren gewählt werden.

Die Erfindung betrifft ferner ein Verfahren zum Betreiben einer Vorrichtung zur drahtlosen Übertragung von Energie zu einem aktiven Implantat und Daten von und/oder zu einem aktiven Implantat, insbesondere wie sie in der vorliegenden Anmeldung beschrieben ist. Das erfindungsgemäße Verfahren kann sämtliche Merkmale aufweisen, die im Zusammenhang mit der erfindungsgemäßen Vorrichtung beschrieben wurden, sowie umgekehrt.

Das erfindungsgemäße Verfahren umfasst die folgenden Verfahrensschritte:
a) Es wird einer Sendespule eine Wechselspannung zugeführt.
b) Eine implantierbare Empfangsspule wird induktiv mit der Sendespule gekoppelt, indem die beiden Spulen in räumlicher Nähe zueinander angeordnet werden. Bevorzugt ist es hierbei, dass die beiden Spulen in axialer Richtung annähernd deckungsgleich angeordnet werden. Hierdurch wird in der Empfangsspule eine Wechselspannung induziert.

Das erfindungsgemäße Verfahren ist gekennzeichnet durch die folgenden Schritte:
c) Eine der implantierten Primärspule zugeführte Wechselspannung wird gemäß einem von der implantierten Primärspule zur extrakorporalen Sekundärspule zu übertragenden Datensignal moduliert.
d) Die modulierte Wechselspannung der implantierten Primärspule induziert in der extrakorporalen Sekundärspule eine modulierte Wechselspannung.
e) Aus dieser wird ein Datensignal extrahiert und ausgewertet. Dies kann durch die bereits beschriebene Auswertschaltung erfolgen.
f) Es werden Informationen betreffend die Energieregelung der von der Sendespule zur übertragenden Energie durch ein pulsweitenmoduliertes Signal von der Primärspule zur Sekundärspule übertragen.
g) Es werden ferner andere Informationen, die nicht die Energieregelung betreffen, durch eine Frequenzmodulation der Überträgerfrequenz des pulsweitenmodulierten Signals oder durch eine Modulation der Frequenz übermittelt, mit der die Pulse des pulsweitenmodulierten Signals gesendet werden.

Die Schritte a) und b) und die Schritte c)-e) müssen aber nicht gleichzeitig ausgeführt werden. Anders ausgedrückt kann eine Datenübertragung auch erfolgen, wenn gerade keine Energie über die Spulen übertragen wird.

Auch das erfindungsgemäße Verfahren kann für einen Datenverkehr in beide Richtungen verwenden.

In einer bevorzugten Ausführungsform erfolgt ein Messen der Signalstärke des von der extrakorporalen Sekundärspule empfangenen Datensignals, so dass basierend hierauf die Qualität der induktiven Kopplung zwischen der Sendespule und der Empfangsspule ermittelt wird.

Im Folgenden wird eine bevorzugte Ausführungsform der Erfindung anhand von Figuren erläutert.

Es zeigen:
- Fig. 1: die grundsätzliche Funktionsweise einer drahtlosen Energieübertragung
- Fig. 2: ein elektrisches Schaltbild einer Ausführungsform der erfindungsgemäßen Vorrichtung.

Fig. 1 wurde bereits im Zusammenhang mit dem Stand der Technik erläutert.

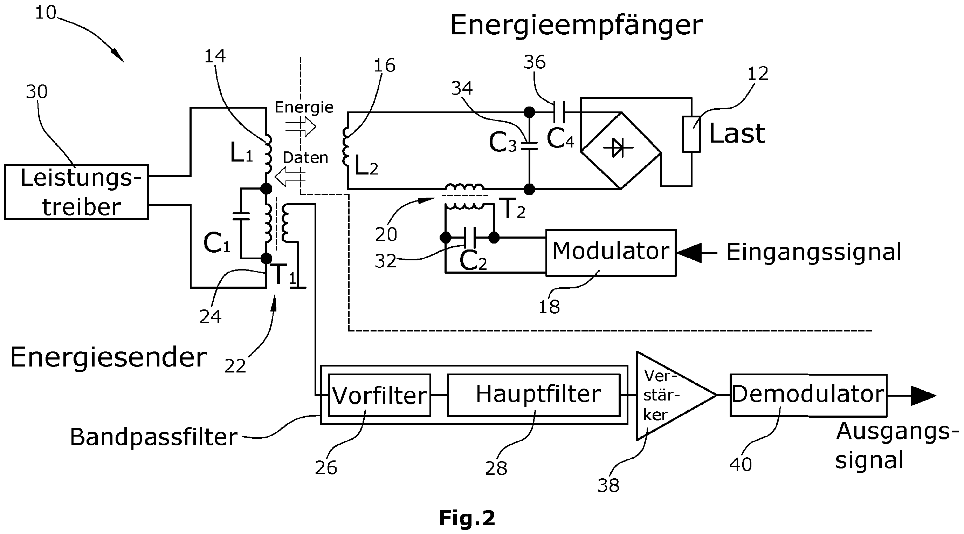

In Fig. 2 ist auf der linken Seite die extrakorporale Sendespule 14 dargestellt. Diese ist mit einem Leistungstreiber 30 zum Einkoppeln der Energie verbunden, die von der Sendespule 14 zur implantierbaren Empfangsspule 16 übertragen werden soll.

Implantatseitig ist auf der rechten Seite der Fig. 2 die Empfangsspule 16 dargestellt. Diese ist mit dem ersten Übertrager 20 verbunden, der eine Primärspule und eine Sekundärspule aufweist. Seine Primärspule ist mit dem Modulator 18 verbunden. Durch diesen erfolgt ein Modulieren einer Wechselspannung gemäß einem von der Empfangsspule 16 zur Sendespule 14 zu übertragenden Datensignals. Die Primärspule dieses Übertragers kann beispielsweise eine Induktivität von ca. 1 µH aufweisen.

Parallel zur Primärspule des Übertragers 20 ist als Resonanzkapazität ein Kondensator 32 geschaltet, der zusammen mit der Primärspule des Übertragers 20 einen auf das Datensignal abgestimmten Parallelschwingkreis ergibt, der den Modulator entlastet.

Mit 34 und 36 sind Abstimmkondensatoren für die Energieübertragung bezeichnet.

Die Empfangsspule 16 überträgt das Datensignal bei einer Frequenz, die deutlich oberhalb der Frequenz für die Energieübertragung liegt. Hierdurch kann sichergestellt werden, dass die Oberwellen der Energieübertragung das Datensignal nicht stören. Beispielsweise kann eine Energieübertragungsfrequenz von 100 kHz und eine Datensignalfrequenz von 455 kHz verwendet werden.

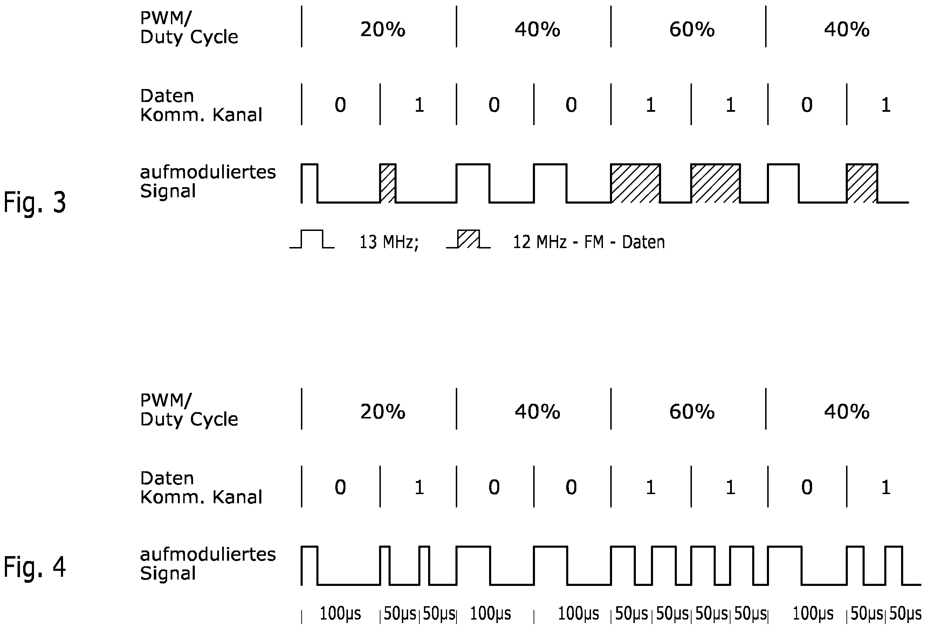

Von der Empfangsspule 16 werden Informationen betreffend die Energieregelung der von der Sendespule 14 zu übertragenen Energie durch ein pulsweitenmoduliertes Signal zur Sendespule 14 übertragen. Ferner werden andere Informationen, die nicht die Energieregelung betreffen, durch eine Frequenzmodulation der Trägerfrequenz des pulsweitenmodulierten Signals oder durch eine Modulation der Frequenz übermittelt, mit der die Pulse des pulsweitenmodulierten Signals gesendet werden. Bei der erstgenannten Variante kann beispielsweise die Trägerfrequenz zwischen 12 Megahertz und 13 Megahertz moduliert werden, wobei beispielsweise die 12 Megahertz einer logischen 1 entsprechen und die 13 Megahertz einer logischen 0. Dies ist in Figur 3 dargestellt.

In der zweiten Variante wird die Frequenz verändert, mit der die Pulse gesendet werden. Hierbei ist es notwendig, dass die beiden verwendeten Frequenzen für eine logische 1 und eine logische 0 das Vielfache voneinander sind. Beispielsweise können die Frequenzen 10 Kilohertz und 20 Kilohertz verwendet werden. Dies bedeutet, dass zwei Pulse mit 20 Kilohertz einer logischen 1 entsprechen und ein Puls mit 10 Kilohertz einer logischen 0. Um parallel hierzu weiterhin eine Energieregelung über die Pulsweitenmodulation zu ermöglichen, wird die Pulsbreite proportional zu der Frequenz (in dem dargestellten Beispiel 10 Kilohertz oder 20 Kilohertz) angepasst, sodass nachwievor der Duty Cycle des pulsweitenmodulierten Signals gleich bleibt. Wird somit eine logische 0 gesendet, so wird lediglich ein Puls mit einer Frequenz von 10 Kilohertz gesendet. Dieser weist die doppelte Pulsbreite auf, wie die zwei Pulse für eine logische 1, die mit einer Frequenz von 20 Kilohertz gesendet werden (in der Annahme, dass in beiden Fällen der gleiche Duty Cycle übertragen werden soll). Diese Ausführungsform ist in Figur 4 dargestellt.

Das von der Empfangsspule 16 gesendete Datensignal wird von der Sendespule 14 empfangen und an den zweiten Übertrager 22 weitergeleitet. Dieser weist eine Primärspule auf, die mit der elektrischen Leitung 24 verbunden ist, die wiederum mit der Sendespule 14 verbunden ist. Diese Primärspule kann eine relativ niedrige Induktivität von beispielsweise 1 µH aufweisen und dient dem Auskoppeln des hochfrequenten Datensignals. Dieses wird sodann einem Vorfilter 26 zugeführt, bei dem es sich vorzugsweise um einen LC-Bandpassfilter handelt. Hierdurch erfolgt ein Begrenzen der Leistungsfrequenz vor dem Einspeisen in den Bandpassfilter.

Das Signal wird sodann dem Bandpassfilter 28 zugeführt, bei dem es sich um einen auf das Datensignal abgestimmten schmalbandigen Filter handelt. Dieser ist vorzugsweise als Keramikfilter ausgelegt.

Hiernach sind ein Verstärker 38 und ein Demodulator 40 angeordnet.

Die Empfangsspule 16 erzeugt somit ein Magnetfeld, das dem zu übermittelnden Datensignal entspricht. Die Sendespule 14 nimmt dieses Magnetfeld auf und erzeugt einen entsprechenden Strom, welcher über den zweiten Übertrager 22 auf die Auswertschaltung gekoppelt wird, die den Vorfilter 26, den Bandpassfilter 28, den Verstärker 38 sowie den Modulator 40 umfasst.

Das Ausgangssignal des Übertragers 22 kann gleichzeitig zur Messung des Primärstroms der Energieübertragung herangezogen werden.

Das Verwenden einer Signalfrequenz von 455 kHz ist besonders vorteilhaft, weil es hierfür sehr schmalbandige Keramikfilter gibt. Außerdem ist es hochfrequent genug, um einen hohen Datendurchsatz zur Verfügung zu stellen.

Die Amplitude des Datensignals kann gleichzeitig als Maß für die Güte der induktiven Kopplung zwischen der Sendespule 14 und der Empfangsspule 16 dienen.

Grundsätzlich kann eine Datenübertragung auch von der Sendespule 14 zur Empfangsspule 16 erfolgen. Hierzu ist es notwendig, auf Seiten der Sendespule 14 einen entsprechenden Modulator sowie die weiteren beschriebenen Komponenten vorzusehen, während implantatseitig eine entsprechende Auswertschaltung vorgesehen sein muss. Die entsprechenden Schaltungsteile müssen somit ausgetauscht werden.

Ebenfalls möglich ist eine bidirektionale Übertragung, wobei hier bevorzugt unterschiedliche Frequenzen verwendet werden.

Weiterhin ist es möglich, in einer Richtung mehrere Frequenzen zu verwenden und damit verschiedene Datenkanäle zu realisieren.

## Patentansprüche

1. Energieübertragungsvorrichtung (10) zur drahtlosen Übertragung von Energie zu einem aktiven Implantat (12), aufweisend
eine extrakorporale Sendespule (14), die mit einer Energiequelle elektrisch verbindbar ist, und
eine implantierbare Empfangsspule (16), die zur drahtlosen Energieübertragung induktiv mit der Sendespule (14) koppelbar ist,
eine extrakorporale Sekundärspule (14);
einen Modulator;
eine implantierbare Primärspule (16), die zur drahtlosen Datenübertragung von der Primärspule zur Sekundärspule induktiv mit der Sekundärspule koppelbar ist,
wobei
die implantierbare Primärspule (16) elektrisch mit dem Modulator (18) verbunden ist, wobei der Modulator (18) ausgestaltet ist, eine der implantierbaren Primärspule (16) zugeführte Wechselspannung gemäß einem Datensignal so zu modulieren, dass eine Datenübertragung von der implantierbaren Primärspule (16) zu der extrakorporalen Sekundärspule (14) erfolgt,
wobei die Frequenz der Datenübertragung verschieden von der Frequenz ist, mit der die Energie von der Sendespule (14) zur Empfangsspule (16) übertragen wird,
wobei das Datensignal Informationen betreffend eine Energieregelung der von der Sendespule (14) zu übertragenden Energie und andere Informationen, die nicht die Energieregelung betreffen, enthält;
**dadurch gekennzeichnet, dass**
die Energieübertragungsvorrichtung ausgestaltet ist, ein Übertragen von Informationen betreffend die Energieregelung der von der Sendespule (14) zu übertragenden Energie durch ein pulsweitenmoduliertes Signal von der Primärspule (16) zur Sekundärspule (14) auszuführen, und dass die Energieübertragungsvorrichtung ausgestaltet ist, ein Übermitteln anderer Informationen, die nicht die Energieregelung betreffen, durch eine Frequenzmodulation der Trägerfrequenz des pulsweitenmodulierten Signals oder durch eine Modulation der Frequenz, mit der die Pulse des pulsweitenmodulierten Signals gesendet werden, auszuführen, und dass
der Modulator (18) durch einen Übertrager (20) induktiv mit einem mit der implantierten Primärspule (16) verbundenen elektrischen Leiter gekoppelt ist.

2. Energieübertragungsvorrichtung nach Anspruch 1, **dadurch gekennzeichnet, dass** die implantierte Primärspule die Empfangsspule (16) ist und/oder die extrakorporale Sekundärspule die Sendespule (14) ist.

3. Energieübertragungsvorrichtung (10) nach Anspruch 1 oder 2, **dadurch gekennzeichnet, dass** die Frequenz der Datenübertragung von der implantierten Primärspule (16) zur extrakorporalen Sekundärspule (14) höher ist als die Frequenz, mit der die Energie von der Sendespule (14) zur Empfangsspule (16) übertragen wird.

4. Energieübertragungsvorrichtung (10) nach einem der Ansprüche 1 bis 3, **gekennzeichnet durch** einen zweiten Übertrager (22) zum induktiven Auskoppeln des von der extrakorporalen Sekundärspule (14) empfangenen Datensignals aus einer mit der extrakorporalen Sekundärspule (14) verbundenen elektrischen Leitung (24).

5. Energieübertragungsvorrichtung (10) nach einem der Ansprüche 1 bis 4, **gekennzeichnet durch** einen Bandpassfilter (28), der lediglich die Datenübertragungsfrequenz passieren lässt.

6. Energieübertragungsvorrichtung (10) nach einem der Ansprüche 1 bis 5, **gekennzeichnet durch** einen zweiten Modulator, der elektrisch mit der extrakorporalen Sekundärspule (14) verbunden ist, zum Modulieren einer der extrakorporalen Sekundärspule (14) zugeführten Wechselspannung gemäß einem von der extrakorporalen Sekundärspule (14) zu der implantierten Primärspule (16) zu übertragenden zweiten Datensignal.

7. Energieübertragungsvorrichtung (10) nach Anspruch 6, **dadurch gekennzeichnet, dass** die für die Datenübertragung von der extrakorporalen Sekundärspule (14) zur implantierten Primärspule (16) verwendete Frequenz verschieden von der für die Datenübertragung von der implantierten Primärspule (16) zur extrakorporalen Sekundärspule (14) verwendete Frequenz ist.

8. Energieübertragungsvorrichtung (10) nach Anspruch 6, **dadurch gekennzeichnet, dass** die für die Datenübertragung von der extrakorporalen Sekundärspule (14) zur implantierten Primärspule (16) verwendete Modulationsverfahren verschieden von dem für die Datenübertragung von der implantierten Primärspule (16) zur extrakorporalen Sekundärspule (14) verwendeten Modulationsverfahren ist.

9. Verfahren zum Betreiben einer Energieübertragungsvorrichtung (10) zur drahtlosen Übertragung von Energie zu einem aktiven Implantat (12), gemäß einem der Ansprüche 1 bis 8, wobei das Verfahren die folgenden Schritte aufweist:
a) Zuführen einer Wechselspannung zu der Sendespule (14),
b) induktives Koppeln der implantierbaren Empfangsspule (16) mit der Sendespule (14), so dass in der implantierbaren Empfangsspule (16) eine Wechselspannung induziert wird,
c) Modulieren der implantierten Primärspule (16) zugeführten Wechselspannung gemäß einem von der implantierten Primärspule (16) zu einer extrakorporalen Sekundärspule (14) zu übertragenden Datensignal, wobei das Datensignal Informationen betreffend eine Energieregelung der von der Sendespule (14) zu übertragenden Energie und andere Informationen, die nicht die Energieregelung betreffen, enthält.
d) Induzieren einer modulierten Wechselspannung in der extrakorporalen Sekundärspule (14) durch die modulierte Wechselspannung der implantierten Primärspule (16),
e) Extrahieren und Auswerten des von der extrakorporalen Sekundärspule (14) empfangenen Datensignals,
**gekennzeichnet durch** die folgenden weiteren Verfahrensschritte:
f) Übertragen von Informationen betreffend die Energieregelung der von der Sendespule (14) zu übertragenden Energie durch ein pulsweiten moduliertes Signals von der implantierten Primärspule (16) zur extrakorporalen Sekundärspule (14),
g) Übermitteln anderer Informationen, die nicht die Energieregelung betreffen, durch eine Frequenzmodulation der Trägerfrequenz des pulsweitenmodulierten Signals oder durch eine Modulation der Frequenz, mit der die Pulse des pulsweitenmodulierten Signals gesendet werden,
h) Messen der Signalstärke des von der extrakorporalen Sekundärspule (14) empfangenen Datensignals, so dass basierend hierauf die Qualität der induktiven Kopplung zwischen der Sendespule (14) und der Empfangsspule (16) ermittelt wird.

10. Verfahren nach Anspruch 9, **dadurch gekennzeichnet, dass** bei einer Modulation der Frequenz, mit der die Pulse des pulsweitenmodulierten Signals gesendet werden, die Pulsbreite proportional zu der Frequenz angepasst wird, mit der die Pulse des pulsweitenmodulierten Signals gesendet werden.

11. Verfahren nach Anspruch 9 oder 10, **dadurch gekennzeichnet, dass** die Trägerfrequenz des pulsweitenmodulierten Signals 1 Megahertz bis 13 Megahertz ist.

12. Verfahren nach einem der Ansprüche 9 bis 11, **dadurch gekennzeichnet, dass** ein Senden der Pulse des pulsweitenmodulierten Signals mit einer Frequenz von 1 Kilohertz bis 20 Kilohertz erfolgt.

13. Verfahren nach einem der Ansprüche 9 - 12, **dadurch gekennzeichnet, dass** das Übermitteln der anderen Informationen ungetaktet, das heißt über einen asynchronen Kommunikationskanal erfolgt.

## Claims

1. An energy transmission device (10) for wireless transmission of energy to an active implant (12), comprising
an extracorporeal transmitter coil (14) electrically connectable to an energy source, and
an implantable receiver coil (16) which can be inductively coupled to the transmitter coil (14) for wireless energy transmission,
an extracorporeal secondary coil (14),
a modulator,
an implantable primary coil (16) which can be inductively coupled to the secondary coil for wireless data transmission from the primary coil to the secondary coil,
wherein
the implantable primary coil (16) is electrically connected to the modulator (18), wherein the modulator (18) is adapted to modulate an AC voltage supplied to the implantable primary coil (16) according to a data signal such that a data transmission from the implantable primary coil (16) to the extracorporeal secondary coil (14) is performed,
wherein the frequency of the data transmission is different than the frequency with which the energy is transmitted from the transmitter coil (14) to the receiver coil (16),
wherein the data signal includes information regarding energy regulation of the energy to be transmitted from the transmitter coil (14) and other information not regarding energy regulation,
**characterized in that**
the energy transmission device is adapted to perform a transmission of information regarding energy regulation of the energy to be transmitted from the transmitter coil (14) by a pulse width modulated signal from the primary coil (16) to the secondary coil (14), and that the energy transmission device is adapted to perform a transmission of other information, not regarding energy regulation, by a frequency modulation of the carrier frequency of the pulse width modulated signal or by a modulation of the frequency with which the pulses of the pulse width modulated signal are sent, and that
the modulator (18) is inductively coupled by a transmitter (20) to an electrical conductor which is connected to the implanted primary coil (16).

2. The energy transmission device according to claim 1, **characterized in that** the implanted primary coil is the receiver coil (16) and/or the extracorporeal secondary coil is the transmitter coil (14).

3. The energy transmission device (10) according to claim 1 or 2, **characterized in that** the frequency of the data transmission from the implanted primary coil (16) to the extracorporeal secondary coil (14) is higher than the frequency with which the energy is transmitted from the transmitter coil (14) to the receiver coil (16).

4. The energy transmission device (10) according to any one of claims 1 to 3, **characterized by** a second transmitter (22) for inductively decoupling the data signal received by the extracorporeal secondary coil (14) from an electrical conductor (24) connected to the extracorporeal secondary coil (14).

5. The energy transmission device (10) according to any one of claims 1 to 4, **characterized by** a band-pass filter (28) which merely allows for the data transmission frequency to pass.

6. The energy transmission device (10) according to any one of claims 1 to 5, **characterized by** a second modulator which is electrically connected to the extracorporeal secondary coil (14) for modulating an AC voltage supplied to the extracorporeal secondary coil (14) according to a second data signal to be transmitted from the extracorporeal secondary coil (14) to the implanted primary coil (16).

7. The energy transmission device (10) according to claim 6, **characterized in that** the frequency used for the data transmission from the extracorporeal secondary coil (14) to the implanted primary coil (16) is different than the frequency used for the data transmission from the implanted primary coil (16) to the extracorporeal secondary coil (14).

8. The energy transmission device (10) according to claim 6, **characterized in that** the modulation methods used for the data transmission from the extracorporeal secondary coil (14) to the implanted primary coil (16) are different than the modulation methods used for the data transmission from the implanted primary coil (16) to the extracorporeal secondary coil (14).

9. A method for operating an energy transmission device (10) for wireless transmission of energy to an active implant (12) according to any one of claims 1 to 8, the method comprising the following steps:
a) supplying an AC voltage to the transmitter coil (14),
b) inductive coupling of the implantable receiver coil (16) to the transmitter coil (14) such that an AC voltage is induced in the implantable receiver coil (16),
c) modulating the AC voltage supplied to the implanted primary coil (16) according to a data signal to be transmitted from the implanted primary coil (16) to an extracorporeal secondary coil (14), wherein the data signal includes information regarding energy regulation of the energy to be transmitted from the transmitter coil (14) and other information not regarding energy regulation,
d) inducing a modulated AC voltage in the extracorporeal secondary coil (14) by the modulated AC voltage of the implanted primary coil (16),
e) extracting and evaluating the data signal received by the extracorporeal secondary coil (14),
**characterized by** the following further steps:
f) transmitting information regarding energy regulation of the energy to be transmitted from the transmitter coil (14) by a pulse width modulated signal from the implanted primary coil (16) to the extracorporeal secondary coil (14),
g) transmitting other information, not regarding energy regulation, by a frequency modulation of the carrier frequency of the pulse width modulated signal or by a modulation of the frequency with which the pulses of the pulse width modulated signal are sent,
h) measuring the signal strength of the data signal received by the extracorporeal secondary coil (14) such that the quality of the inductive coupling between the transmitter coil (14) and the receiver coil (16) is determined based thereon.

10. The method according to claim 9, **characterized in that**, in case of a modulation of the frequency with which the pulses of the pulse width modulated signal are sent, the pulse width is adjusted proportionally to the frequency with which the pulses of the pulse width modulated signal are sent.

11. The method according to claim 9 or 10, **characterized in that** the carrier frequency of the pulse width modulated signal is 1 megahertz to 13 megahertz.

12. The method according to any one of claims 9 to 11, **characterized in that** the pulses of the pulse width modulated signal are sent with a frequency of 1 kilohertz to 20 kilohertz.

13. The method according to any one of claim 9 to 12, **characterized in that** the transmission of the other information is performed in a non-clocked manner, i.e. via an asynchronous communication channel.

## Revendications

1. Dispositif de transmission de puissance (10) destiné à transmettre sans fil une puissance à un implant actif (12), comprenant :
un bobinage émetteur extracorporel (14), lequel peut être relié électriquement à une source de puissance, et
un bobinage récepteur implantable (16), lequel peut être en mis en couplage inductif avec le bobinage émetteur (14) pour la transmission de puissance sans fil,
un bobinage secondaire extracorporel (14) ;
un modulateur ;
un bobinage primaire implantable (16) lequel peut être en mis en couplage inductif avec le bobinage secondaire pour la transmission sans fil de données depuis le bobinage primaire vers le bobinage secondaire,
dans lequel
le bobinage primaire implantable (16) est relié électriquement au modulateur (18), dans lequel le modulateur (18) est configuré pour moduler une tension alternative apportée au bobinage primaire implantable (16) selon un signal de données de telle sorte qu'une transmission de données s'effectue depuis le bobinage primaire implantable (16) vers le bobinage secondaire extracorporel (14),
dans lequel la fréquence de la transmission de données est différente de la fréquence avec laquelle la puissance est transmise depuis le bobinage émetteur (14) vers le bobinage récepteur (16),
dans lequel le signal de données contient des informations concernant une régulation de la puissance à transmettre depuis le bobinage émetteur (14) et d'autres informations ne concernant pas la régulation de puissance ;
**caractérisé en ce que**
le dispositif de transmission de puissance est configuré pour effectuer une transmission d'informations concernant une régulation de la puissance à transmettre depuis le bobinage émetteur (14) par le biais d'un signal modulé en largeur d'impulsion depuis le bobinage primaire (16) vers le bobinage secondaire (14), et **en ce que** le dispositif de transmission de puissance est configuré pour effectuer une communication d'autres informations ne concernant pas la régulation de puissance, par le biais d'une modulation de fréquence de la fréquence porteuse du signal modulé en largeur d'impulsion ou par le biais d'une modulation de la fréquence avec laquelle les impulsions du signal modulé en largeur d'impulsion sont émises, et **en ce que**
le modulateur (18) est couplé par induction à un conducteur électrique relié au bobinage primaire implanté (16) par le biais d'un transmetteur (20).

2. Dispositif de transmission de puissance selon la revendication 1, **caractérisé en ce que** le bobinage primaire implanté est le bobinage récepteur (16) et/ou le bobinage secondaire extracorporel est le bobinage émetteur (14).

3. Dispositif de transmission de puissance (10) selon la revendication 1 ou 2, **caractérisé en ce que** la fréquence de la transmission de données depuis le bobinage primaire implanté (16) vers le bobinage secondaire extracorporel (14) est supérieure à la fréquence avec laquelle la puissance est transmise depuis le bobinage émetteur (14) vers le bobinage récepteur (16).

4. Dispositif de transmission de puissance (10) selon l'une des revendications 1 à 3, **caractérisé par** un deuxième transmetteur (22) pour le découplage inductif du signal de données reçu du bobinage secondaire extracorporel (14) depuis un conducteur électrique (24) relié au bobinage secondaire extracorporel (14).

5. Dispositif de transmission de puissance (10) selon l'une des revendications 1 à 4, **caractérisé par** un filtre passe-bande (28), lequel laisse passer uniquement la fréquence de transmission de données.

6. Dispositif de transmission de puissance (10) selon l'une des revendications 1 à 5, **caractérisé par** un deuxième modulateur, lequel est relié électriquement au bobinage secondaire extracorporel (14), afin de moduler une tension alternative apportée au bobinage secondaire extracorporel (14) selon un deuxième signal de données à transmettre depuis le bobinage secondaire extracorporel (14) vers le bobinage primaire implantable (16).

7. Dispositif de transmission de puissance (10) selon la revendication 6, **caractérisé en ce que** la fréquence utilisée pour la transmission de données depuis le bobinage secondaire extracorporel (14) vers le bobinage primaire implanté (16) est différente de la fréquence utilisée pour la transmission de données depuis le bobinage primaire implanté (16) vers le bobinage secondaire extracorporel (14) .

8. Dispositif de transmission de puissance (10) selon la revendication 6, **caractérisé en ce que** le procédé de modulation utilisé pour la transmission de données depuis le bobinage secondaire extracorporel (14) vers le bobinage primaire implanté (16) est différent du procédé de modulation utilisé pour la transmission de données depuis le bobinage primaire implanté (16) vers le bobinage secondaire extracorporel (14).

9. Procédé de fonctionnement d'un dispositif de transmission de puissance (10) destiné à transmettre sans fil une puissance à un implant actif (12) selon l'une des revendications 1 à 8, dans lequel le procédé comprend les étapes suivantes :
a) apport d'une tension alternative au bobinage émetteur (14),
b) couplage inductif du bobinage récepteur implantable (16) avec le bobinage émetteur (14), de sorte qu'une tension alternative est induite dans le bobinage récepteur implantable (16),
c) modulation de la tension alternative apportée au bobinage primaire implanté (16) selon un signal de données à transmettre depuis le bobinage primaire implanté (16) vers un bobinage secondaire extracorporel (14), dans lequel le signal de données contient des informations concernant une régulation de la puissance à transmettre depuis le bobinage émetteur (14) et d'autres informations ne concernant pas la régulation de puissance.
d) induction d'une tension alternative modulée dans le bobinage secondaire extracorporel (14) par la tension alternative modulée du bobinage primaire implanté (16),
e) extraction et contrôle du signal de données reçu par le bobinage secondaire extracorporel (14),
**caractérisé par** les étapes de procédé supplémentaires suivantes :
f) transmission d'informations concernant la régulation de la puissance à transmettre depuis le bobinage émetteur (14) par le biais d'un signal modulé en largeur d'impulsion depuis le bobinage primaire implanté (16) vers le bobinage secondaire extracorporel (14),
g) communication d'autres informations ne concernant pas la régulation de puissance, par le biais d'une modulation de fréquence de la fréquence porteuse du signal modulé en largeur d'impulsion ou par le biais d'une modulation de la fréquence avec laquelle les impulsions du signal modulé en largeur d'impulsion sont émises,
h) mesure de l'intensité du signal de données reçu par le bobinage secondaire extracorporel (14), de sorte qu'à partir de cette mesure la qualité du couplage inductif entre le bobinage émetteur (14) et le bobinage récepteur (16) est déterminée.

10. Procédé selon la revendication 9, **caractérisé en ce que** lors d'une modulation de la fréquence avec laquelle les impulsions du signal modulé en largeur d'impulsion sont émises, la largeur d'impulsion est adaptée proportionnellement à la fréquence, avec laquelle les impulsions du signal modulé en largeur d'impulsion sont émises.

11. Procédé selon la revendication 9 ou 10, **caractérisé en ce que** la fréquence porteuse du signal modulé en largeur d'impulsion est comprise entre 1 et 13 mégahertz.

12. Procédé selon l'une des revendications 9 à 11, **caractérisé en ce qu'**une émission de l'impulsion du signal modulé en largeur d'impulsion s'effectue avec une fréquence comprise entre 1 et 20 kilohertz.

13. Procédé selon l'une des revendications 9 à 12, **caractérisé en ce que** la communication des autres informations s'effectue de manière non-synchronisée, c'est-à-dire par le biais d'un canal de communication asynchrone.
